# EUROPEAN PATENT APPLICATION

(11) **EP 4 024 404 A1**
(43) Date of publication of application: **06.07.2022**
(21) Application number: 21150024.4
(22) Date of filing: 04.01.2021
(51) Int. Cl.: G16H 40/60, A61B 6/00

(54) **METHOD AND SYSTEM FOR CONTROLLING MEDICAL IMAGING SESSIONS AND OPTIMIZING DISCOMFORT OF THE PATIENT**

(71) Applicant: Koninklijke Philips N.V., 5656 AG Eindhoven (NL)
(72) Inventor: LEUSSLER, Christoph Günther, 5656 AE Eindhoven (NL); WUELBERN, Jan Hendrik, 5656 AE Eindhoven (NL); VOGTMEIER, Gereon, 5656 AE Eindhoven (NL); WIESS, Steffen, 5656 AE Eindhoven (NL); JOHNSON, Mark, 5656 AE Eindhoven (NL); SISODIA, Rajendra Singh, 5656 AE Eindhoven (NL)
(74) Representative: Philips Intellectual Property & Standards

(57) **Abstract**

The present invention relates to a method and a system (100) for controlling medical imaging sessions, the system (100) comprising a medical imaging parameter generator (10), which is configured to generate a set of parameters for a medical imaging scan of a medical imaging device (1000) based on patient input data at least partially obtained during a preparatory scan, the patient input data comprising at least one threshold level with regard to a at least one medical imaging workflow condition initiating at least one discomfort to the patient; and a medical imaging workflow controller (20), which is configured to control the medical imaging device (1000) based on the set of parameters and which is configured to optimize an impact of the initiated at least one discomfort to the patient for the controlling of the medical imaging device (1000).

## Description

### FIELD OF THE INVENTION

The present invention relates to a method and a system for controlling medical imaging sessions and for optimizing discomfort, to a method of training a machine-learning model, to a computer program element, and to a computer readable medium.

### BACKGROUND OF THE INVENTION

Standard magnetic resonance, MR, imaging sessions as well as medical imaging sessions in general are performed under full control of experienced staff members. They position the patient on the MR patient support, apply sensors as an ECG unit for cardiac scans, select, adapt and run the specific set of scans, perform some immediate image quality control, archive the images, and dismiss the patient from the MR suite. Because MR sessions are typically relatively long, this represents a large cost factor in radiology. Therefore, more autonomous imaging approaches are desirable, in which at least some of the above steps are automatized. Other drivers towards autonomous imaging are generally increasing numbers of MR examinations and a lack of experienced staff in many regions.

Typical MR imaging is accompanied with high acoustic noise, thermal heating of the patient and peripheral nerve stimulation, PNS, of the muscles. The sensation and reaction on these parameters depend on the individual patient. Often the scans for medical imaging sessions in general are interrupted due to strong discomfort of the patient, as the scans need to run for a relative long time.

### SUMMARY OF THE INVENTION

There may therefore be a need for improved medical imaging systems. The object of the present invention is achieved by the subject matter of the independent claims where further embodiments are incorporated in the dependent claims. It should be noted that the following described implementations of the aspects of the invention equally applies to the system and the method, to a method of training a machine-learning model, to a computer program element, and to a computer readable medium.

According to a first aspect of the invention there is provided a system for controlling medical imaging sessions, the system comprising a medical imaging parameter generator, which is configured to generate a set of parameters for a medical imaging scan of a medical imaging device. The generation of the set of parameters is based on patient input data at least partially obtained during a preparatory scan, the patient input data comprising at least one threshold level with regard to at least one medical imaging workflow condition initiating at least one discomfort to the patient. The system further comprises a medical imaging workflow controller, which is configured to control the medical imaging device based on the set of parameters and which is configured to optimize an impact of the initiated at least one discomfort to the patient for the controlling of the medical imaging device.

The present invention in contrast to the prior art allows that the adaptation of the imaging parameters can be partly realized during patient preparation outside the Magnetic Resonance Imaging, MRI, building, by Apps, or by patient information and patient history.

The present invention in contrast to the prior art allows that the real sensation of acoustic noise, peripheral nerve stimulation, thermal heating by gradient coils in combination inside a small bore or enclosed with coils and sensors need to be realized inside the MRI system. The present invention in contrast to the prior art allows that the sensation of acoustic noise, peripheral nerve stimulation, thermal heating by gradient coils can also be simulated accurately in a multi-sensor simulated environment with virtual reality setup.

The present invention advantageously uses an autonomous demo or system configuration mode, where the patient can interactively optimize an individual level of system parameters he can support over the time of the sequence. The short test helps to actively engage the patient. Thus, the patient when providing feedback and having the chance or option thereto is more concentrated and cooperative.

The present invention in contrast to the prior art allows that the test can be interleaved by or combined with MR system preparation testing.

The present invention advantageously uses co-operation of the patient in an autonomous setup, for instance via a feedback and monitoring method. The present invention advantageously provides that the patient overcomes and resolves any discomfort as for instance anxiety, pain, scare, turn nasty during sequence.

According to an exemplary embodiment of the present invention, the medical imaging workflow controller is configured to optimize the controlling of the medical imaging device by minimizing at least one time period for applying the at least one medical imaging workflow condition.

According to an exemplary embodiment of the present invention, the patient input data further comprises data on at least one time period threshold for the at least one time period for applying the at least one medical imaging workflow condition.

According to an exemplary embodiment of the present invention, the medical imaging workflow is configured to control the medical imaging device for the preparatory scan in at least one of demonstration mode, simulation mode or a geometry planning mode.

According to an exemplary embodiment of the present invention, the at least one medical imaging workflow condition is ramped up either continuously or as in a discrete pattern.

According to an exemplary embodiment of the present invention, the at least one threshold level relates to a maximum tolerable discomfort of the patient, wherein the at least one threshold level is adjustable.

According to an exemplary embodiment of the present invention, the patient input data obtained during a preparatory scan at least further comprises a second threshold level with regard to a second medical imaging workflow condition initiating a second discomfort to the patient, wherein the medical imaging workflow controller is configured to control the medical imaging device based on the set of parameters and is configured to optimize a combined impact of the initiated first discomfort and the initiated second discomfort to the patient for the controlling of the medical imaging device by determining a first time period for the first medical imaging workflow condition and a second time period for the second medical imaging workflow condition based on the set of parameters.

According to an exemplary embodiment of the present invention, a neural network is integrated in the medical imaging workflow controller and the neural network is configured to optimize the impact of the initiated at least one discomfort to the patient for the controlling of the medical imaging device.

According to an exemplary embodiment of the present invention, the medical imaging workflow controller is configured to adjust the medical imaging workflow to move from the first medical imaging workflow condition initiating a first discomfort to the second medical imaging workflow initiating the second discomfort in the patient.

The present invention in advantageous manner and according to an exemplary embodiment allows that a medical imaging workflow is optimized, wherein a preparatory scan is performed which is non-imaging in nature, and wherein the set of parameters used for the (optionally entire) preparatory scan are incapable of producing an image, and wherein the parameters of the preparatory scan are defined such that the sources of discomfort are initiated in a sequence with as little as possible time between them.

There are for instance according to an exemplary embodiment of the present invention at least two factors, which may be taken into account when planning such a sequence:
The response time of the patient to discomfort and the time required by the patient for them to assess their threshold level; and
The technical ability of the system to move from one (extreme) condition initiating a first discomfort to a second (extreme) condition initiating a second discomfort in the patient.

According to an exemplary embodiment of the present invention, with the power of hundreds and thousands of systems analyzed, an artificial intelligence is able to produce predictions that are much more precise than human prediction based on experience only. What is proposed herein is, in exemplary embodiments of the present invention, is based on a regression neural network that derives numerical parameters from a given medical imaging workflow.

In another aspect there is provided a method for providing an autonomous operation mode for medical imaging sessions, the method comprising the following steps,

Generating a set of parameters for a medical imaging scan of a medical imaging device based on patient input data at least partially obtained during a preparatory scan, the patient input data comprising at least one threshold level with regard to a at least one medical imaging workflow condition initiating at least one discomfort to the patient; and

Controlling the medical imaging device based on the set of parameters; and Optimizing an impact of the initiated at least one discomfort to the patient for the controlling of the medical imaging device.

In another aspect still, there is provided a computer program element, which, when being executed by at least one processing unit, is adapted to cause a method to perform the method according to the previous aspect.

In another aspect still, there is provided a neural network trained by the method as described above.

In another aspect still, there is provided a method of training the neural network of the system as described above.

In another aspect still, there is provided at least one computer readable medium having stored thereon the program element, or having stored thereon the machine learning module.

In general, the *"machine learning"* may include a programmed classification based on algorithm that build a model based on sample data, known as "training data", in order to make predictions or decisions without being explicitly programmed to do so.

Some machine learning algorithms are model-based. A model based ML algorithm operates to adjust parameters of a machine learning model. This adjustment procedure is called "training".

The model is thus configured by the training to perform the task. ML algorithms also include instance-based learning. Task performance by the ML algorithm improves measurably, the more new training data is used in the training. The performance may be measured by objective tests when feeding the system with test data. The performance may be defined in terms of a certain error rate to be achieved for the given test data.

### BRIEF DESCRIPTION OF THE DRAWINGS

Exemplary embodiments of the invention will now be described with reference to the following drawings, which, unless stated otherwise, are not to scale.
Fig. 1 shows parallel processes of table position, system scan and patient demo/configuration-training according to an exemplary embodiment of the present invention;
Fig. 2 shows an algorithm sequence of individual threshold tuning of the scan sequence and a demo mode sequence comprising different tuning parameters to be adapted for patient comfort according to an exemplary embodiment of the present invention;
Fig. 3 shows a block diagram of a computer implemented system for controlling medical imaging sessions according to an exemplary embodiment of the present invention;
Fig. 4 shows a flow chart of a method for controlling medical imaging sessions according to an exemplary embodiment of the present invention; and
Fig. 5 shows different selection of threshold level according to an exemplary embodiment of the present invention.

### DETAILED DESCRIPTION OF EMBODIMENTS

The illustration in the drawings is schematically and not to scale. In different drawings, similar or identical elements are provided with the same reference numerals.

Generally, identical parts, units, entities or steps are provided with the same reference symbols in the figures.

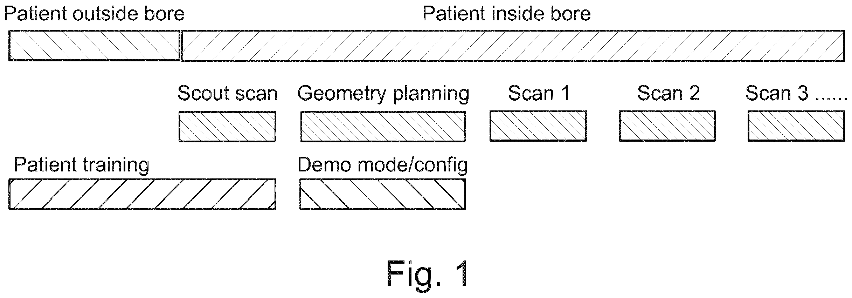

Fig. 1 shows parallel processes of table position, system scan and patient demo mode or configuration training mode according to an exemplary embodiment of the present invention.

According to an exemplary embodiment of the present invention, a system and workflow is provided with an autonomous integrated demo/simulation mode. The patient can actively decide about his individual threshold level he can support, for instance in terms of heating, PNS, acoustic noise, duration of phases of immobilization, mechanical vibrations due to resonance phenomena occurring in parts of the coil or of the patient bed or a component of the imaging system. In other words, any emission of the medical imaging system that induces discomfort to the patient or any structural component inducing discomfort to the patient.

According to an exemplary embodiment of the present invention, the configuration mode is mostly run during preparation and geometrical planning such that minimal extra time is required for the total scan. Due to the individual configured scan parameters, the risk of scan interruption or abort is reduced. It may be possible that the threshold level may change after a scan or a session. The threshold level of the patient may change due to the fact that the patient improves trust and perceives discomfort differently. Then a new threshold level can be determined and further scans be adapted.

According to an exemplary embodiment of the present invention, the demo mode or the simulation mode (acoustic noise & sound melody, PNS, SAR Heating) changes the scan parameters of the MR sequence in a way, so that the patient can select different options. Feedback of patient is provided by sensing techniques (contactless 3D sensing,..). The proposed method empowers the patient and improves his trust to control the system.

According to an exemplary embodiment of the present invention, when the patient is outside the scanner bore, the patient training starts with explanation of the upcoming demo/configuration mode. The training is stopped, when the scout scan is finished.

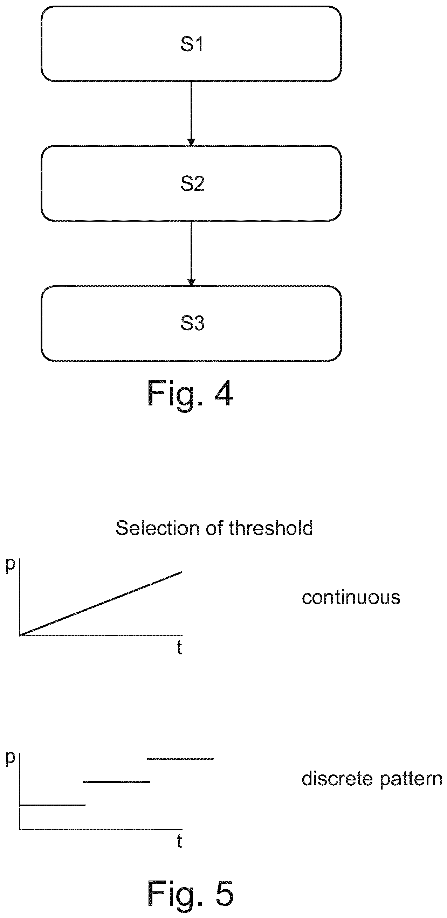

According to an exemplary embodiment of the present invention, during geometry planning no scan is active. During that time interval the demo mode and/or configuration mode is started. The different system parameters are ramped up either continuously or as in a discrete pattern as shown in Fig. 5.

According to an exemplary embodiment of the present invention, the patient can determine the threshold he can support and signal this maximum sustainable level to the system or operator. For example, when the demo mode or configuration mode is finished, the determined system parameters are loaded and configure the following imaging scans.

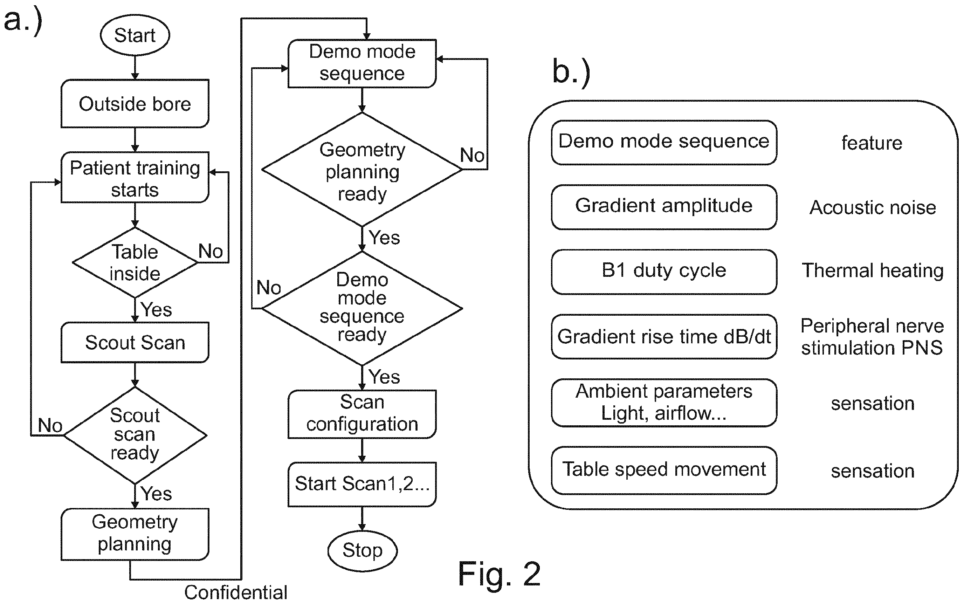

Fig. 2 shows according to an exemplary embodiment of the present invention the algorithm sequence of individual threshold tuning of the scan sequence and the demo mode sequence comprising different tuning parameters to be adapted for patient comfort.

According to an exemplary embodiment of the present invention, the demo-mode is a software sequence, which interactively changes system parameters, which directly influence the patient comfort in the scanner. According to an exemplary embodiment of the present invention, the demo-mode is used to define response times of the patient.

According to an exemplary embodiment of the present invention, the parameters can be run successively or partly in combination. The parameters can be ramped up in a linear or other function - exponential, polynomial or analytic function -, so that the patient can effectively determine the threshold he can support.

According to an exemplary embodiment of the present invention, there are several factors which need to be taken into account when planning such a sequence:
i) The response time of the patient to discomfort and the time required by the patient for them to assess their threshold level. Specifically, it is known that as the sensorial systems of the patients are stimulated these may become insensitive if stimulated for too long. Therefore in preferred embodiments is will be better to plan the demo-mode scan in such a manner to switch between stimuli which cause discomfort in different sensorial systems. For example, both PNS and patient heating provide similar sensations in the tactile system of the patient.

Therefore, in preferred sequences it is preferred not to plan PNS threshold testing scans adjacent to patient thermal heating threshold testing scans. A preferred sequence would be to first start with PNS threshold testing scans, then move to scans to assess the audio threshold (as this is a different sensorial system) and only then proceed to scans to assess the patient heating threshold. Such a sequence gives the patients tactile sensorial nerve system a chance to recover from PNS before moving to the heating sequences.
ii) The technical ability of the system to move from one (extreme) condition initiating a first discomfort to a second (extreme) condition initiating a second discomfort in the patient. This will lead to embodiments where demo-mode sequence will not be chosen which the machine will take a long time to set up. Let us assume that it takes considerable time to switch a machine between PNS threshold testing scans and Heating threshold testing scans. In this example, it is preferred that heating threshold testing scans and sound threshold testing scans can be induced with a first demo-mode sequence (as these are assumed to be easy to transition between) whilst in another part of the demo-mode sequence another part of the sound threshold testing scan sequence is easily transitioned into a PNS threshold testing scan sequence. This would be technically beneficial, for example putting less stress onto the system components, reducing power requirements, or leading to even faster demo-mode sequences etc.

According to an exemplary embodiment of the present invention, the demo-mode software sequence determines with the input from the patient the individual system parameters and communicates with the MRI sequencer. The parameters are transmitted to the MR sequencer and an optimizer software, SW, program (artificial intelligence, AI,-based) calculates the final adapted parameters for the imaging sequence.

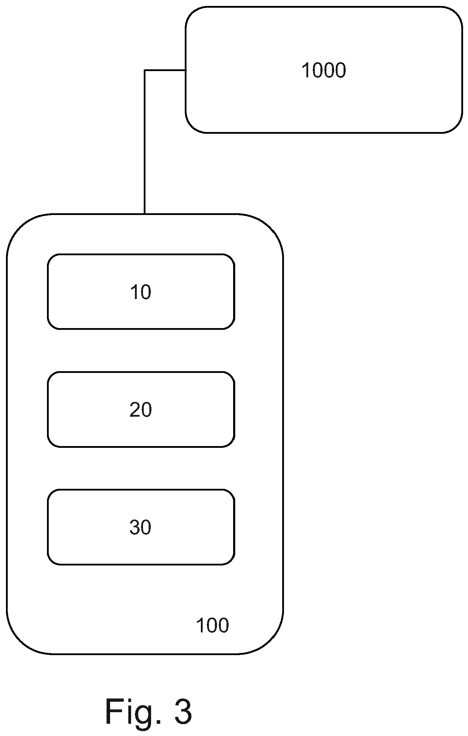

Fig. 3 shows a block diagram of a computer implemented system for controlling medical imaging sessions according to an exemplary embodiment of the present invention. Fig. 3 also shows a block diagram of a medical imaging system according to an exemplary embodiment of the present invention.

The system 100 for controlling medical imaging sessions comprises a medical imaging parameter generator 10 and a medical imaging workflow controller 20.

The medical imaging parameter generator 10 is configured to generate a set of parameters for a medical imaging scan of a medical imaging device 1000 based on patient input data at least partially obtained during a preparatory scan, the patient input data comprising at least one threshold level with regard to a at least one medical imaging workflow condition initiating at least one discomfort to the patient

According to an exemplary embodiment of the present invention, the medical imaging parameter generator 10 is configured to generate the set of parameters based on patient input data at least partially obtained during a preparatory scan, the patient input data comprising a first threshold level and a first response time of the patient with regard to a first medical imaging workflow condition initiating a first discomfort to the patient and a second threshold level and a second response time of the patient with regard to a second medical imaging workflow condition initiating a second discomfort to the patient.

According to an exemplary embodiment of the present invention, the response time only is provided between the first and the second (i.e. wait for second until effect of first has expired away) second response is only needed if a third condition is tested.

The medical imaging workflow controller 20 is configured to control the medical imaging device 1000 based on the set of parameters and which is configured to optimize an impact of the initiated at least one discomfort to the patient for the controlling of the medical imaging device

According to an exemplary embodiment of the present invention, the medical imaging workflow controller 20 is configured to control the medical imaging device 1000 based on set of parameters and which is configured to optimize a combined impact of the initiated first discomfort and the initiated second discomfort to the patient for the controlling of the medical imaging device 1000 by determining a first time period for the first medical imaging workflow condition and a second time period for the second medical imaging workflow condition based on the set of parameters.

According to an exemplary embodiment of the present invention, the system 100 further comprises an input interface 30, configured to receive user input, wherein the first threshold level and/or the second threshold level and/or any further i-th threshold level is adjustable based on the user input.

Fig. 4 shows a flow chart of a method for controlling medical imaging sessions according to an exemplary embodiment of the present invention.

The method for controlling medical imaging sessions comprises the following steps.

At first, generating S1 a set of parameters for a medical imaging scan of a medical imaging device 1000 is performed based on patient input data at least partially obtained during a preparatory scan, the patient input data comprising at least one threshold level with regard to a at least one medical imaging workflow condition initiating at least one discomfort to the patient.

Secondly, controlling S2 the medical imaging device 1000 based on the set of parameters is performed.

Finally, optimizing S3 an impact of the initiated at least one discomfort to the patient for the controlling of the medical imaging device

According to an exemplary embodiment of the present invention, the step of optimizing further comprises optimizing a combined impact of the initiated first discomfort and the initiated second discomfort to the patient for the controlling of the medical imaging device by determining a first time period for the first medical imaging workflow condition and a second time period for the second medical imaging workflow condition based on the set of parameters is conducted.

Fig. 5 shows different selection of threshold level according to an exemplary embodiment of the present invention. Threshold level can be selected from a continuous ramp or from a set of proposed discrete parameter settings

According to an exemplary embodiment of the present invention, the diagnostic system comprises a demo/simulation mode, acoustic noise & sound melody, PNS, SAR Heating, ambient parameters, duration of phases of immobilization, or the like.

According to an exemplary embodiment of the present invention, the patient takes action to decide about limit of sequence parameters. A sequence is started with smooth parameters and values are increased sequentially up to an individual perceived threshold. This threshold is taken to calculate parameters of the listed scans.

According to an exemplary embodiment of the present invention, the sequence can start with maximum levels which are decreased until a patient sustainable level is found. According to an exemplary embodiment of the present invention, the threshold may can also be determined in combination with a relative person - pediatric application - in the scanner room.

According to an exemplary embodiment of the present invention, the threshold level is interactively transmitted via sensors.

According to an exemplary embodiment of the present invention, the parameters can be set by autonomous software or by cloud-based systems or by embedded systems or by remote operator.

According to an exemplary embodiment of the present invention, a successful scan session and improved patient experience is provided.

According to an exemplary embodiment of the present invention, the further active settings are to be configured: a diagnostic system can be configured during demo-mode regarding coil fitting, adaptive mattress, ambient lightning, air flow etc.

According to an exemplary embodiment of the present invention, the system is configured to adapt environmental parameters (light, sequence, entertainment, distraction, personal assistance) based on a threshold.

According to an exemplary embodiment of the present invention, the system is configured to induce a table movement, for instance a speed or an acceleration.

According to an exemplary embodiment of the present invention, the system is configured to provide feedback sensing between patient and system.

According to an exemplary embodiment of the present invention, the system is configured to generate a simulating environment with multi-sensory output and reproducing other input via a virtual reality setup

According to an exemplary embodiment of the present invention, the system is configured to remote nudging to indicate that the table will start/stop moving - patient can move a bit when the table moves but should be stationary during scan.

According to an exemplary embodiment of the present invention, the system is configured to that when the patient takes action, the system overrides active control, reaction. In another embodiment the patient can take control and actively decides about the next steps - e.g. when the next scan could start depending on the comfort level of the patient.

According to an exemplary embodiment of the present invention, the system is configured to determine that a decision to do the next step could be trained and tested before in the simulation run which helps the patient to get a good understanding of what will happen during the scan.

According to an exemplary embodiment of the present invention, classification of threshold values with respect to patient data - e.g. size of the patient, weight of the patient, (pathologic) history of the patient - and apply for fixed system start values or start parameters for different (remote) MRI scanners.

According to an exemplary embodiment of the present invention, the individual comfort and/or pain level of the patient then is the guideline to take action for the start / stop / continuation of the scan. The simulation mode shows the exact planning and illustrates what the patient has to expect and with that is important feedback from the system to the patient already before the scanning procedure. This information includes the overall scan time the number of scans and the expected impact on the patient for the individual subscan. This experience allows the patient to make a prognosis of his stress level and the breaks he might need before continuation with the next scan.

The patient can influence the start / stop / continue already during the simulation mode and with this functionality gains trust in the system that the final exam would be done as the patient expects. The positive feeling about "having control" and the "personal readiness" has impact on the scan speed and quality.

User interfaces for the patient-scanner interaction could be manifold and several technologies like, simple switches, speech control, gesture control, movement control and more could be used. This includes the communication patient to scanner but also the communication scanner to patient might use these user interfaces. Additional displaying devices and information sharing technologies would complete the technical realization.

According to an exemplary embodiment of the present invention, the demo/ simulation mode is used to determine the individually acceptable duration of phases of immobilization of the patient, which is used to adapt scan parameters accordingly. In a related embodiment, the patient is animated to move between such phases/scans or in phases where motion does not disturb MR scanning. The scanner may therefore issue automated audio commands or video commands. The scanner may detect this intentional patient motion with sensors as the MR system itself, cameras, or other suitable sensors. As soon as the patient stops moving the scanner automatically announces continuation of the scan and asks the patient to remain immobilized for the next scan.

This approach will improve patient experience in at least two ways. Firstly, patients frequently have difficulties to lie still for a long period, and this approach offers the possibility to relax with some movements from time to time. Secondly, the approach empowers the patient to control the MR exam. Technically, patients will immobilize better as required during actual scanning which will improve image quality and shorten scan times.

During the demo/simulation mode the patient is trained to move and immobilize himself as requested.

According to an exemplary embodiment of the present invention, the alternate way of generating a simulated/demo mode is outside of the machine room so as to enable the use of setup for scans and not hold it for simulation demo mode.

In this setup, a multi-sensory environment is created - for instance a multi sensory system for e.g. immersive display that has the ability to simulate generating multi-sensor output - such as heat, surround sound, light as well as other PNS. This are coupled with a VR system, that can also provide the patient with other input such as an experience of sliding into a actual MR room.

According to an exemplary embodiment of the present invention, the multi-sensory input is then coupled with the patient information system, the scan sequence simulation system so that the actual output of sound, PNS, heat, immobility input can be generated.

According to an exemplary embodiment of the present invention, the setup can be part of patient preparation/training in the workflow so that a patient wear a VR headset which has the actual MR room visualization. The VR environment is enclosed in actual multi-sensory system that simulates actual sequence, vibration, heat and other PNS sensor so that the response of the patient can be determined.

The advantage of the above described embodiment of the present invention is that the demo mode can be experienced for the scout scan, partial scan or full scan.

One or more features described herein can be configured or implemented as or with circuitry encoded within a computer-readable medium, and/or combinations thereof. Circuitry may include discrete and/or integrated circuitry, a system-on-a-chip (SOC), and combinations thereof, a machine, a computer system, a processor and memory, a computer program.

In another exemplary embodiment of the present invention, a computer program or a computer program element is provided that is characterized by being adapted to execute the method steps of the method according to one of the preceding embodiments, on an appropriate system.

The computer program element might therefore be stored on a computer unit, which might also be part of an embodiment of the present invention. This computing unit may be adapted to perform or induce a performing of the steps of the method described above. Moreover, it may be adapted to operate the components of the above-described apparatus. The computing unit can be adapted to operate automatically and/or to execute the orders of a user. A computer program may be loaded into a working memory of a data processor. The data processor may thus be equipped to carry out the method of the invention.

This exemplary embodiment of the invention covers both, a computer program that right from the beginning uses the invention and a computer program that by means of an up-date turns an existing program into a program that uses the invention.

Further on, the computer program element might be able to provide all necessary steps to fulfill the procedure of an exemplary embodiment of the method as described above.

According to a further exemplary embodiment of the present invention, a computer readable medium, such as a CD-ROM, is presented wherein the computer readable medium has a computer program element stored on it which computer program element is described by the preceding section.

A computer program may be stored and/or distributed on a suitable medium (in particular, but not necessarily, a non-transitory medium), such as an optical storage medium or a solid-state medium supplied together with or as part of other hardware, but may also be distributed in other forms, such as via the internet or other wired or wireless telecommunication systems.

However, the computer program may also be presented over a network like the World Wide Web and can be downloaded into the working memory of a data processor from such a network. According to a further exemplary embodiment of the present invention, a medium for making a computer program element available for downloading is provided, which computer program element is arranged to perform a method according to one of the previously described embodiments of the invention.

It has to be noted that embodiments of the invention are described with reference to different subject matters. In particular, some embodiments are described with reference to method type claims whereas other embodiments are described with reference to the device type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

While the invention has been illustrated and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. A single processor or other unit may fulfill the functions of several items re-cited in the claims.

The mere fact that certain measures are re-cited in mutually different dependent claims does not indicate that a combination of these measures cannot be used to advantage. Any reference signs in the claims should not be construed as limiting the scope of the claims.

### LIST OF REFERENCE SIGNS:

- 10: Generator
- 20: Medical imaging workflow controller
- 30: Input interface
- 100: System
- 1000: Medical imaging device
- S1: Generating
- S2: Controlling
- S3: Optimizing

## Claims

1. A system (100) for controlling medical imaging sessions, the system (100) comprising
- a medical imaging parameter generator (10), which is configured to generate a set of parameters for a medical imaging scan of a medical imaging device (1000) based on patient input data at least partially obtained during a preparatory scan, the patient input data comprising at least one threshold level with regard to a at least one medical imaging workflow condition initiating at least one discomfort to the patient; and
- a medical imaging workflow controller (20), which is configured to control the medical imaging device (1000) based on the set of parameters and which is configured to optimize an impact of the initiated at least one discomfort to the patient for the controlling of the medical imaging device (1000).

2. The system of claim 1, wherein the medical imaging workflow controller (20) is configured to optimize the controlling of the medical imaging device (1000) by minimizing at least one time period for applying the at least one medical imaging workflow condition.

3. The system of claim 2, wherein the patient input data further comprises data on at least one time period threshold for the at least one time period for applying the at least one medical imaging workflow condition.

4. The system of any one of previous claims, wherein the medical imaging workflow is configured to control the medical imaging device (1000) for the preparatory scan in at least one of demonstration mode, simulation mode or a geometry planning mode.

5. The system of any one of previous claims, wherein the at least one medical imaging workflow condition is ramped up either continuously or as in a discrete pattern.

6. The system of any one of previous claims, wherein the at least one threshold level relates to a maximum tolerable discomfort of the patient, wherein the at least one threshold level is adjustable.

7. The system of any one of previous claims, wherein the patient input data obtained during a preparatory scan at least further comprises a second threshold level with regard to a second medical imaging workflow condition initiating a second discomfort to the patient, wherein the medical imaging workflow controller (20) is configured to control the medical imaging device (1000) based on the set of parameters and is configured to optimize a combined impact of the initiated first discomfort and the initiated second discomfort to the patient for the controlling of the medical imaging device (1000) by determining a first time period for the first medical imaging workflow condition and a second time period for the second medical imaging workflow condition based on the set of parameters, wherein optionally the patient input data further comprises at least one first response time of the patient with regard to the first medical imaging workflow condition and wherein optionally at least one second response time of the patient with regard to the second medical imaging workflow condition.

8. The system of any one of previous claims, wherein a neural network is integrated in the medical imaging workflow controller (20) and the neural network is configured to optimize the impact of the initiated at least one discomfort to the patient for the controlling of the medical imaging device (1000).

9. The system of any one of previous claims, wherein the medical imaging workflow controller (20) is configured to adjust the medical imaging workflow to move from the first medical imaging workflow condition initiating a first discomfort to the second medical imaging workflow initiating the second discomfort in the patient.

10. A method of training the neural network of the system according to any one of claims 8 to 9.

11. A neural network trained by the method according to claim 10.

12. A medical imaging device comprising a system of any one of the previous claims.

13. A method for controlling medical imaging sessions, the method comprising the following steps,
- Generating (S1) a set of parameters for a medical imaging scan of a medical imaging device (1000) based on patient input data at least partially obtained during a preparatory scan, the patient input data comprising at least one threshold level with regard to a at least one medical imaging workflow condition initiating at least one discomfort to the patient; and
- Controlling (S2) the medical imaging device (1000) based on the set of parameters; and
- Optimizing (S3) an impact of the initiated at least one discomfort to the patient for the controlling of the medical imaging device (1000).

14. A computer program element, which, when being executed by at least one processor is adapted to cause the processor to perform the method according to claim 13.

15. At least one computer readable medium having stored thereon the program element according to claim 14.
